# EUROPEAN PATENT APPLICATION

(11) **EP 4 124 865 A1**
(43) Date of publication of application: **01.02.2023**
(21) Application number: 21188288.1
(22) Date of filing: 28.07.2021
(51) Int. Cl.: G01N 33/569

(54) **A SURROGATE CELL-BASED CORONA VIRUS SPIKE PROTEIN BLOCKING ASSAY**

(71) Applicant: Friedrich-Alexander-Universität Erlangen-Nürnberg, 91054 Erlangen (DE)
(72) Inventor: MIELENZ, Dirk Alexander, 91054 Erlangen (DE); JÄCK, Hans-Martin, 91088 Bubenreuth (DE)
(74) Representative: Grünecker Patent- und Rechtsanwälte PartG mbB

(57) **Abstract**

To monitor infection by the severe acute respiratory syndrome coronavirus 2 (SARS-CoV-2) and successful vaccination against coronavirus disease 2019 (COVID-19), the kinetics of neutralizing or blocking anti-SARS-CoV-2 antibody titers need to be assessed. Here, we report the development of a quick and inexpensive surrogate SARS-CoV-2 blocking assay (SUBA) using immobilized recombinant human ACE-2 (hACE-2, ACE-2) and human cells expressing the native form of surface SARS-CoV-2 spike protein. Spike protein-expressing cells bound to hACE-2 in the absence or presence of blocking antibodies were quantified by measuring the optical density of cell-associated crystal violet in a spectrophotometer. The advantages are that SUBA is a fast and inexpensive assay which does not require biosafety level 2- or 3-approved laboratories. Most importantly, SUBA detects blocking antibodies against the native trimeric cell-bound SARS-CoV-2 spike protein and can quickly be adjusted to quickly pre-screen already approved therapeutic antibodies or sera from vaccinated individuals for their ACE-2 blocking activities against any emerging SARS-CoV-2 variants.

## Description

### Background of the invention

Coronavirus disease 19 (COVID-19) caused by the severe acute respiratory syndrome coronavirus 2 (SARS-CoV-2) is a highly infectious disease with variable outcome. Typical symptoms are fever, sore throat, cough, myalgia, as well as neurological and gastrointestinal symptoms (1, 2). Especially advanced age, high blood pressure, diabetes or obesity are risk factors for a severe disease. A fraction of patients develops severe pneumonia requiring intensive care. If lung function is severely compromised, those patients require artificial ventilation or, at worst, extra corporal blood oxygenation (1-3). SARS-CoV-2 infection as well as mRNA-, DNA vector- and protein-based vaccinations induce neutralizing antibodies and antigen-specific T cells (4-14). Both, vaccination and infection induce memory B and T cells (15-17). Most - but not all - neutralizing antibodies interfere with the interaction of the receptor binding domain (RBD) of the trimeric SARS-CoV-2 spike protein with the Angiotensin-converting enzyme (ACE-2), its cognate host receptor (18-21).

To monitor a SARS-CoV-2 infection and successful treatments and vaccination strategies, the serum titers of neutralizing or blocking anti-SARS-CoV-2 antibodies must be assessed and quantified. The gold standards are virus neutralization assays with pseudo-typed or infectious SARS-CoV-2 using Vero cells (22) or plaque-reduction neutralization assays (23). These assays require not only the infectious replication-competent SARS-CoV-2, pseudo-typed retroviruses or vesicular stomatitis virus expressing the SARS-CoV-2 spike protein (24) but also biosafety level 3 or 2 (25), respectively, and the precise titration of the multiplicity of infection (MOI) for each viral preparation (14).

SARS-CoV-2 blocking antibodies can also be quantified by competitive ELISA assays using recombinant and labeled trimeric ecto spike protein (S_{ecto}) or receptor binding domain (RBD) proteins (26, 27). However, commercial assays using immobilized recombinant trimeric SARS-CoV-2 spike protein are expensive and the industrial production of recombinant proteins to detect emerging SARS-CoV-2 variants is time-consuming. Besides, a recombinant protein barely reflects the spike protein's native configuration as given on the virus and could be denatured during non-compliant storage. Most importantly, existing test systems to assay blocking antibodies should be quickly adaptable to escape mutants that appear to be more infectious and are suspected to worsen the disease (14, 28-30). Existing surrogate virus neutralization tests (39-40) make use of recombinant spike proteins, either attached to a surface or added in soluble form to human ACE-2 attached to a surface. They thus suffer from high safety requirements (require a biosafety level 2 facility) and uncertainty regarding the conformation of the corona virus spike protein (or corona spike protein) in the test. EP 3800473 A1 is an example of such a surrogate virus neutralization test.

There is, thus, a need for an accurate, cheap, easy to distribute, and safe test system for assaying agents capable of blocking the interaction between SarsCoV-2 and its cellular targets.

### Summary of the invention

The present inventors were able of establishing a test system and assay format fulfilling these demands.

Provided are means and methods useful for identifying blocking agents and neutralizing inhibitors, such as neutralizing antibodies of a corona virus infection.

Further provided is a cell useful in the methods of the present invention. The test system and assay format of the invention is universal and can be used to identify blocking agents, such as neutralizing antibodies, for other viral infections as well.

The test system, assay format and the methods of the present invention provide
a) very good means for comparison of the tests between different laboratories,
b) a simple and easy to distribute means for the test, since the spike protein-producing cells of the present invention can be easily shipped,
c) an accurate test, since the spike-producing cells produce the spike protein in the same native conformation as given on the virus and this is achieved in any laboratory in the world under standard culture conditions,
d) a safe test, since no S2 or S3 laboratory (and thus no time-consuming and costly application for approval by the authorities) is necessary,
e) a safe and cheap test, since costly virus production, storage and titration are no longer necessary,
e) a safe test, since the costly production of recombinant, trimeric spike proteins or mutant spike proteins or components thereof (e.g. receptor binding domain, RBD) is no longer necessary
f) a simple and reliable test, no longer requiring approval of the native conformation as it occurs on the virus,
g) economic advantages due to the detection of the bound cells, which is carried out via a simple staining with a cheap and quasi unlimited dye (such as crystal violet),
h) elimination of expensive detection devices, and
i) good comparability between different laboratories, since no enzymatic detection reactions are necessary.

The present invention is capable of tracking and characterizing antibodies blocking the binding of SARS-CoV-2 spike protein to its cognate ACE-2 receptor ("ACE-2", "hACE-2" or "human ACE-2", herein), in a simple, inexpensive and reproducible surrogate cell-based SARS-CoV-2 spike protein blocking assay (SUBA). SUBA can be performed under standard biosafety level 1 conditions in any molecular biology laboratory, and most importantly, it utilizes the native, membrane-bound form of the SARS-CoV-2 spike protein.

### Embodiments of the invention are

1. A method of assaying a compound for its ability to block the binding of SARS-CoV-2 to its cellular target, preferably of the corona virus spike protein to the human ACE-2 receptor, the method comprising:
a) incubating human ACE-2 with:
   i) a ligand, which is a corona virus spike protein that has the ability to bind with specificity to human ACE-2;
   ii) said compound;
b) determining the amount of the ligand bound to said human ACE-2;
c) comparing the results obtained in step b) with those obtained when incubating human ACE-2 with the ligand but in the absence of said compound; and
d) concluding that said compound blocks the binding of the corona virus spike protein to human ACE-2, if the amount of binding observed in the presence of said compound is lower than in the absence of said compound.

Preferably, the corona virus spike protein ("corona spike protein", "spike protein" and spike" are synonyms herein) is in its native conformation; i.e. presents the same epitopes as presented on the corona virus capsid. "Blocking" the binding of the corona virus spike protein to human ACE-2 means "inhibiting" the binding of the corona virus spike protein to human ACE-2.

To "bind with specificity" means that cells expressing spike bind only to ACE-2 coated plates and not to non-coated plates, cells not expressing spike do not bind to plates coated with ACE-2. The binding of spike-expressing cells to ACE-2 is saturable as a function of the ACE-2 coating concentration, the expression of spike on the cell surface and the concentration of the cells in the ACE-2 coated wells.

The compound ("compound" and "test compound" are equivalents herein) may be an antibody, small molecule and/or anti-receptor protein.

In a further aspect, the test compound may be a potentially therapeutically active substance derived from a fungus, an animal or human, e.g. from a serum of an animal or human.

In another aspect, the test compound may be a synthetic compound, e.g. a biomimetic.

In still another aspect, the test compound may be a serum of a patient or a compound in or isolated from a serum of a patient.

It is further contemplated that the test compound may be a medicament, e.g. a medicament comprising a therapeutic antibody.

The term "human ACE-2" (or "ACE-2") includes human ACE-2 as complete receptor for the virus or as a functional fragment thereof, which is capable of binding to the spike protein of the SARS-CoV-2, e.g. the ectodomain of human ACE-2.

2. The method of item 1, wherein the human ACE-2 is immobilized to a surface, such as a membrane, or is immobilised on a solid support.

According to the present invention, surfaces or solid supports are selected from surfaces established in analytics. Typical examples are well plates or isolated membranes of cells expressing on their surface ACE-2 with the membranes being attached to well plates.

3. The method of any of items 1-2, wherein the corona virus spike protein is expressed on the surface of a cell.

Preferably, cells are not immobilized on a surface, in particular if ACE-2 is immobilized; e.g. cells are in aqueous suspension. In a different assay format, cells may be immobilized if ACE-2 is not immobilized]

Preferably, according to the present invention, the concentration of cells is 0.01 - 1 x 10⁷ cells/ml, more preferably 0.5-4 x 10⁶ cells /ml. A typical assay volume is 10 - 1000 µl, preferably 100 µl per assay.

4. The method of any of items 1-3, wherein the corona virus spike protein is expressed on the surface of a cell at a protein density between 1 -100 x 10⁴ corona virus spike protein molecules per cell.

Preferably, the corona virus spike protein is expressed on the surface of a cell at a protein density between 5- 20 x 10⁴ corona virus spike protein molecules per cell.

In another aspect, the amount of corona virus spike protein per cell determined as weight per cell is between 0.25 -25 x 10⁻¹⁴ g, preferably 1.2 -4.8 x 10⁻¹⁴ g .

5. The method of any of items 1-4, wherein the corona virus spike protein is the spike protein of SARS-CoV2, preferably the spike protein comprising SEQ ID NO:1, or a spike protein exhibiting 90% identity with SEQ ID NO:1.

The corona virus spike protein may be a spike protein from one of the seven known human Coronavirus Species (HCoV) from two Genera: Alpha-coronaviruses (HCoV-229E, HCoV-NL63) and Beta-coronaviruses (HCoV-HKU1, HCoV-OC43, SARS-CoV, MERS-CoV, SARS-CoV-2).

In another aspect, the corona virus spike protein may be from a SARS-CoV-2 variant; a variant may be a variant occurring due to mutation, e.g. alpha, beta, delta variant of SARS-CoV-2. Preferably, the corona virus spike protein is the spike protein of a SARS-CoV2 consensus sequence for alpha, beta, gamma and delta variant, more preferably the spike protein comprising SEQ ID NO:5, or a spike protein exhibiting 90%, most preferably 98%, identity with SEQ ID NO:5.

The term "spike protein" or "spike" encompasses the full complete spike protein as well as functional fragments thereof having the ability to specifically bind with human ACE-2, e.g. S1-domain ("S1"), S2-domain("S2"), and a protein comprising receptor binding domain ("RBD") of the spike protein.

The receptor human ACE-2 is the same for all SARS coronaviruses. Other Coronaviruses besides SARS may target other receptors. Therefore, other coatings than ACE-2 may be used, if Spike proteins from other coronaviruses or other viruses will be analyzed.

6. The method of any of items 1-5, wherein said cells expressing the corona virus spike protein on their surface are cells that have been transfected with a vector comprising a promoter operably linked to a sequence coding for the corona virus spike protein.

Preferably, the cells are prepared by a recombinant technique to incorporate the corona virus spike protein, e.g. by transfection (of adherent cells or in suspension), ecotropic retroviral transduction or lentiviral transduction.

7. The method of any of items 4-6, wherein said cells expressing the corona virus spike protein are detectably labelled.

Preferably, the label is introduced into the cells before or after the binding of human ACE-2 to the corona virus spike protein.

According to one aspect of the invention, the label may be a native enzyme present in the cell, e.g. hexosamidase; or may be a protein label, e.g. green fluorescent protein (GFP) or derivates thereof (such as BFP etc.), e.g. incorporated by transfection or infection via a recombinant technique. Other synthetic labels or dye molecules are also envisaged.

The label may be incorporated into cells or attached to the cell surface. The label may be attached by staining of DNA or protein. Examples of labels are crystal violet, propidium iodide or Coomassie blue.

In one aspect, the label may be attached to the test compound after binding; e.g. by a secondary antibody against a test compound which is an antibody.

In another aspect, labelling may be such, that label is detected on a bound ACE-2 - spike protein complex or the label is attached to unbound ACE-2 or unbound spike protein, i.e. ACE-2 and spike protein not attached to each other.

In another aspect, labelling may make use of a competitive reaction.

8. The method of item 7, wherein the label is a dye, fluorphore, or a fluorochrome.

9. The method of any of item 8, wherein the label is crystal violet, or coomassie blue,or green fluorescent protein, or propidium iodide or any other fluorescent dye.

The label may stain proteins, nucleic acids and/or carbohydrates.

10. The method of any of items 7-9, wherein the label is incorporated into the cells expressing the corona virus spike protein.

Incorporation can be achieved by staining cells with dyes after fixation and/or permeabilization, or by transfection or infection of cells with a fluorescent protein produced by recombinant techniques. After incorporation, the label is contained in the cell.

The detection of the label is preferably as follows: The cells are lysed to release the label into a medium, preferably an aqueous medium; more preferably, the label can be detected via optical detection, e.g. using a photometer; OD (i.e. optical density) may be detected and quantified. 11. A host cell expressing a corona virus spike protein on its surface,

Preferably the cell presents the corona virus spike protein in its native conformation; more preferably, the cell is a mammalian cell, or a yeast cell, or single cell algae.

Even more preferably the cell is a Ramos B cell, HEK 293.

The corona virus spike protein expressed on the cell surface may be a corona virus spike protein as defined in the method of any of items 1-10.

The cells may be prepared by a technique to incorporate the corona virus spike protein, e.g. by transfection (of adherent cells or in suspension), ecotropic retroviral transduction or lentiviral transduction.

The cells can be conserved and/or stored by freezing in freezing medium at -70°C; preferably long term storage comprises cooling at - 180°C.

Cells can be transfered to customers. The customers may cultivate cells for use; cells are cultivated under standard conditions. Preferably, cells may be transferred to customers upon mild fixation in dead form, still allowing the surrogate binding assay.

12. The host cell of item 11, further including a label.

The label may be a dye, fluorochrome or fluorophore; enzymatic label; radiolabel; etc. The label may be any label defined in the methods of the present invention. 13. Use of the host cell of item 11 or 12 in a surrogate coronavirus neutralization test.

The corona virus spike protein may be a corona virus spike protein as used in the method of any of items 1-10.

The cell is preferably used to present the corona antigen and/or potential mutations thereof that recognize neutralizing antibodies by blocking the interaction of the corona virus spike protein with the human ACE-2 receptor.

14. A transfection vector comprising a nucleic acid sequence encoding an amino acid sequence of a corona virus spike protein.

Prefarably, the transfection vector comprises a nucleic acid sequence encoding an internal ribosomal entry site (IRES) coupled to GFP or any other fluorescent protein and/or an enzyme, such as hexosaminidase or beta galactosidase.

The corona virus spike protein may be any corona virus spike protein as defined in the method of any of items 1-10]

15. A method of assaying a test compound for its ability to block the binding of a viral surface protein to a cell receptor, comprising:
a) incubating the cell receptor with:
   i) a ligand, which is a viral surface protein that has the ability to bind with specificity to the cell receptor;
   ii) said test compound;
b) determining the amount of the ligand bound to said cell receptor;
c) comparing the results obtained in step b) with those obtained for an incubated cell receptor incubated with the ligand but in the absence of said test compound; and
d) concluding that said test compound blocks the binding of the viral surface protein to the cell receptor, if the amount of binding observed in the presence of said test compound is lower than in the absence of said test compound.

Preferably, the viral surface protein is in its native conformation. Preferably, the cell receptor is a human cell receptor. If the viral infection may occur in an animal, the cell receptor may be an animal cell receptor. "Blocking" the binding of the viral surface protein to the cell receptor means "inhibiting" the binding of the viral surface protein to the cell receptor.

Examples of the virus in the above embodiment are:
- The virus is HIV and the viral surface protein is gp120
- The virus is a SARS-Coronavirus (SARS-CoV-1) or a MERS virus and the viral surface protein is the spike of SARS-CoV-1 or a MERS virus
- The virus is any other pathogenic human virus with a known receptor
- The virus is SARS-CoV-2 and the cell receptor is human ACE-2
- The virus is a virus infecting an animal, preferably a mammal, more preferably a human patient.

All preferred embodiments of items 2-14 apply *mutatis mutandis* to the method of item 15.

16. A method of analysing a sample for the presence of a compound capable of inhibiting and/or neutralizing corona virus infection, comprising:
a) contacting the sample with the corona virus spike protein as cited in any of items 1-11, and the humanACE-2 protein as cited in any of items 1-11, and
b) determining the level of interaction between the corona virus spike protein and the ACE-2.

A reduced interaction indicates the presence of an inhibiting compound.

17. The method according to item 16, wherein the sample is
a) a blood sample, a lymph sample, a saliva sample, or a synovial fluid sample; or
b) the sample comprises a compound to be tested for inhibiting activity.

The test compound may be an antibody, a small molecule, and/or an anti-receptor protein (i.e. a receptor binding protein).

The test compound may be a potentially therapeutically active substance derived from a fungus or an animal, e.g. from a serum, or sputum or faeces or any other body fluid.

The test compound may be a synthetic compound, e.g. a biomimetic.

The test compound may be a serum of a patient or a compound in or isolated from a serum of a patient.

The test compound may be a medicament, e.g. a medicament comprising a therapeutic antibody.

The human ACE-2 is as defined for items 1-14 and 16.

18. A method for diagnosis of an infection with a corona virus, comprising
a) providing a sample previously collected from a subject to be tested;
b) contacting the sample with the spike protein as cited in any of items 1-11 and the human ACE-2 protein as cited in any of items 1-11; and
c) determining the level of interaction between the spike protein and the human ACE-2;
wherein the reduction of the interaction level in the presence of the sample indicates the subject is infected or was infected with a corona virus.

19. The method according to any of items 16-18, wherein the corona virus is SARS-CoV-2 or a variant thereof.

20. Kit comprising the cell of item 11 or 12 together with a recombinant purified receptor protein.

A "purified cell receptor", "purified receptor" or "purified receptor protein" is a purified receptor targeted by a viral protein.

Preferably, the kit comprises a label as defined in any of items 1-19 above.

21. A method of preparing an inhibitor of the binding of a viral surface protein to a cell receptor comprising
a) identifying the inhibitor by performing the method of any of items 1-10 or 15, and
b) isolating the inhibitor.

### Figures

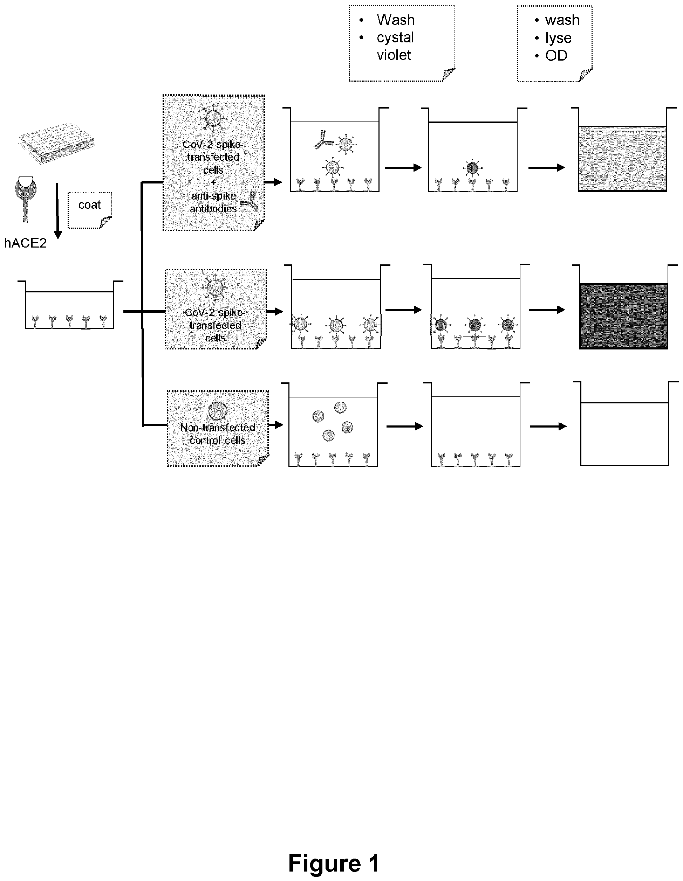

Figure 1 shows the Design of the method and the surrogate SARS-CoV-2 spike blocking assay (SUBA) of the present invention.
   A solid support, e.g. 96-well plates, is coated with soluble recombinant human ACE-2. Cells, such as Ramos B cells or HEK293T cells, expressing the corona virus spike protein, e.g. constitutively or tetracycline-inducibly expressing the SARS-CoV-2 spike protein, are attached to the solid support in the absence or presence of anti-spike protein compounds, e.g. anti-spike protein antibodies. One possible mode of detection is shown: Non-bound cells are removed; bound cells are fixed and stained with a dye, here crystal violet. Fixed crystal violet is solubilized and quantified by detecting its optical signal intensity in a US-Vis spectrophotometer. The higher the optical density, the more cells are bound to fixed ACE-2, which means that less inhibitory compound is in the analytic sample.

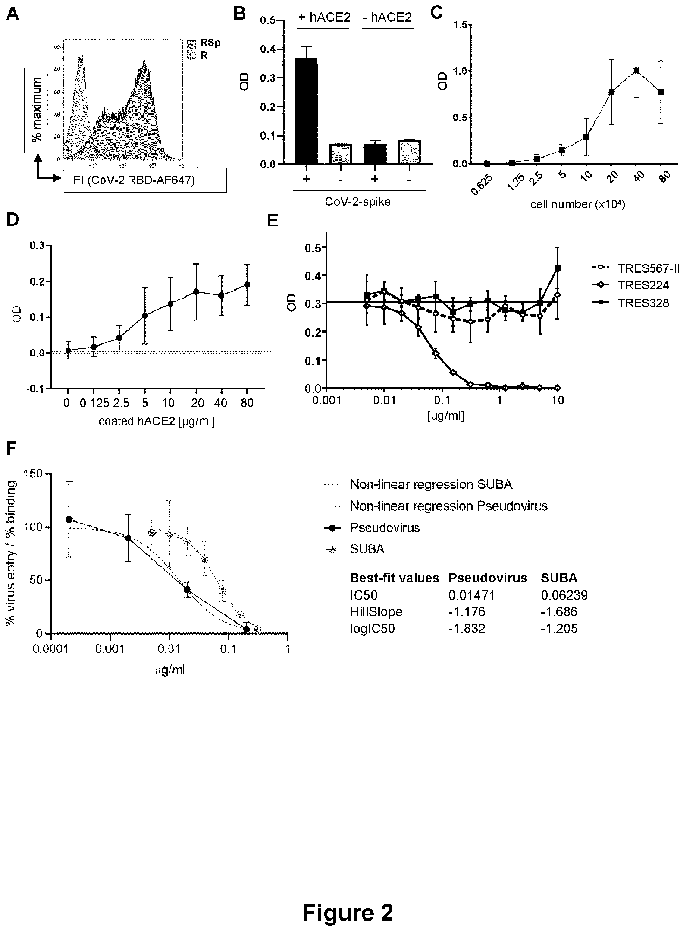

Figure 2 shows the Determination of various parameters of the methods of the present invention
   A Stably spike-expressing Ramos-null B cells (RSp) or Ramos-null B cells (R) were stained with the recombinant anti-SARS CoV-2-spike-RBD binding antibody TRES224, followed by secondary AF647-conjugated anti-human IgG antibody and analyzed by flow cytometry. B 10⁵ Stably spike-expressing Ramos-null B cells (RSp) or Ramos-null B cells (R) were allowed to attach to hACE-2- or non-coated plates, fixed and stained with crystal violet. Crystal violet was solubilized and OD₅₇₀ₙₘ was measured. Data are depicted as mean -/+ SD of triplicate samples. C Titration of the cell number per 96 well with stably SARS-CoV-2-spike expressing Ramos-null B cells (RSp) and background (Ramos-null B cells) subtracted. Data are presented as mean -/+ SD of three experiments performed with each three replicates. D Titration of the hACE-2 coating concentration (µg/ml) with stably SARS-CoV-2-spike-expressing Ramos-null B cells (RSp; 10⁵/well) and background (Ramos-null B cells) subtracted. Data are presented as mean -/+ SD of three experiments performed with each three replicates. E Blockage of RSp cell binding to hACE-2 with the RBD-specific TRES224 antibody. 10⁵ RSp cells were allowed to attach to hACE-2-coated plates in the presence of increasing concentrations of the anti-RBD neutralizing antibody TRES224, an anti-SARS-CoV-2-spike N-terminal domain (NTD) antibody (TRES328) and an isotype-matched control antibody (TRES567-II), fixed and stained with crystal violet. Crystal violet was solubilized and OD₅₇₀ₙₘ was measured. The black line represents the control (without antibody). Data are represented as mean -/+ SD of four measurements from two experiments. F The anti-RBD neutralizing antibody TRES224 was tested in a virus-neutralization assay (mean -/+ SD of four replicates) and in SUBA (2 experiments performed with each three replicates, mean -/+ SD). Data were fitted by non-linear regression and IC50 values as well as Hill slopes are depicted.

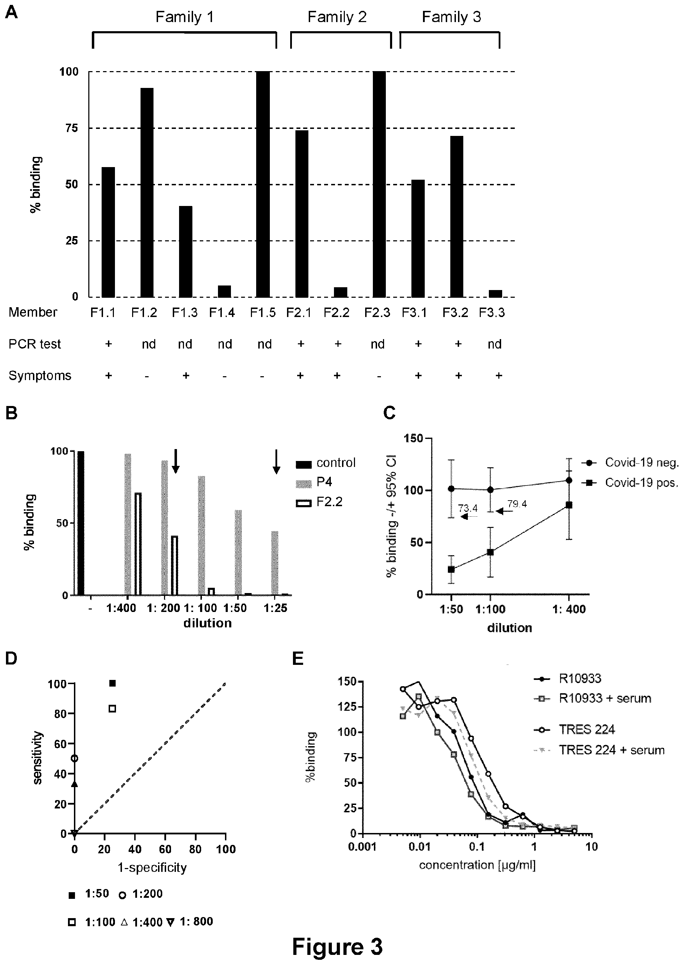

Figure 3 shows the Analysis of spike-blocking activity in human sera.
   **A** Spike-expressing Ramos-null B cells (RSp; 10⁵/well) were allowed to attach to hACE-2-coated wells in the presence or absence of sera of purified SARS-CoV-2 blocking antibodies or sera from SARS-CoV-2 infected individuals, with (+) or without (-) symptoms, from three families (F1-F3) with at least one SARS-CoV-2 PCR-positive family member (PCR test: +). Plates were washed, fixed, stained with crystal violet, solubilized and OD was measured. The background (Ramos-null B cells) was subtracted. Data are presented as mean % binding relative to control (RSp cells and ACE-2 coated wells in the absence of serum or blocking antibodies) of three measurements performed in triplicates each. nd: not determined. B The assay was performed with increasing dilutions of sera with no (control), weak (serum P4) and strong blocking activity (serum F2.2). Spike-expressing Ramos-null B cells (RSp; 10⁵/well) were allowed to attach to hACE-2-coated wells in the presence or absence of different dilutions or sera with previously defined weak or strong blocking activity. Data are presented as mean % binding relative to control (RSp cells in the absence of serum or blocking antibodies) performed in triplicate. Red arrows indicate the dilutions where similar blocking activity was detected. C. Sera from 8 COVID-19 negative and 6 COVID-19 positive donors were subjected to SUBA. Data are depicted as mean -/+ 95% CI. Red arrows and numbers depict the lower limit of COVID-19 negative sera. **D.** *Receiver operating characteristic* (ROC) analysis of SUBA using the data obtained in C and additional serum dilutions. **E** Spike-expressing Ramos-null B cells (RSp; 10⁵/well) were allowed to attach to hACE-2-coated wells in the presence of increasing concentrations of the monoclonal antibodies TRES224 or Regeneron R10933 diluted either with the assay medium or a human serum from an individual tested negative for anti-SARS-CoV-2 antibodies. Data are presented as mean % binding relative to control (RSp cells in the absence of serum or blocking antibodies) performed in triplicates.

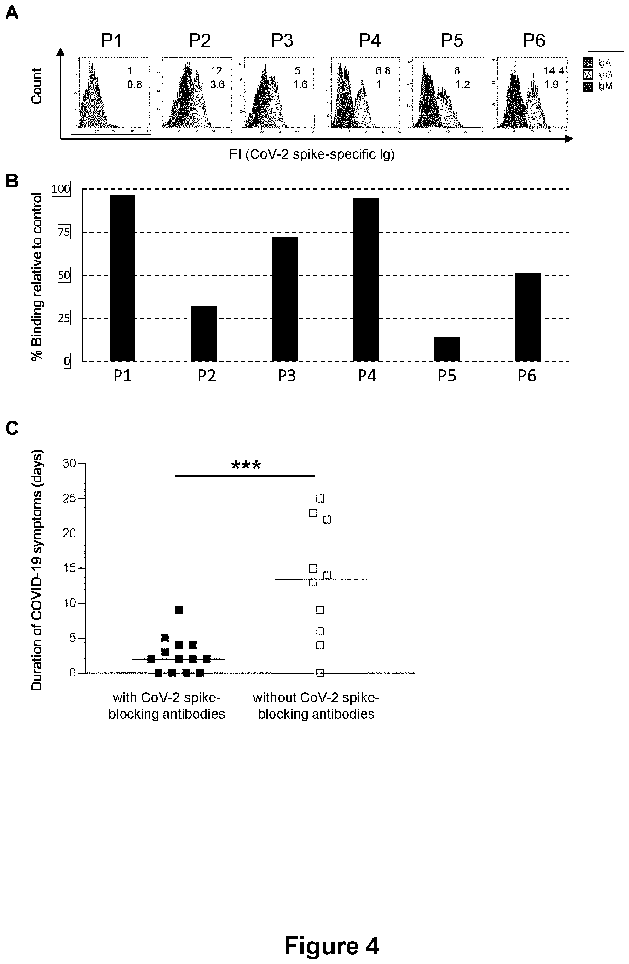

Figure 4 exhibits the Analysis of SARS-CoV-2 spike-binding and -blocking antibodies.
   **A** Flow cytometric analysis of SARS-CoV-2 spike-binding IgM, IgA and IgG serum antibodies in patients (P1-P6) from different families with at least one PCR-positive case of SARS-CoV-2 infection. P1 served as a negative control and did not contain serum antibodies recognizing the SARS-CoV-2 spike protein. HEK293T cells were transiently transfected with a plasmid encoding SARS-CoV-2 spike protein and stained with sera as described in Figure S2. Numbers indicate the mean fluorescence intensities (MFI) of SARS-CoV-2-Spike IgG (green) and IgA (blue). **B** SUBA assay to detect SARS-CoV-2-blocking serum antibodies in sera from patients P1-P6. Data are presented as mean % RSp binding relative to control (RSp cells in the absence of serum or blocking antibodies) of three measurements performed in triplicates each. **C** Correlation between CoVID-19 symptoms and the presence of SARS-CoV-2 spike-blocking antibodies in family members with at least one reported PCR-positive SARS-CoV-2 infection. Typical symptoms were fever, head and body ache, diarrhea, exhaustion, cough, shortness of breath, chest tightness. p = 0.0003 (unpaired t test).

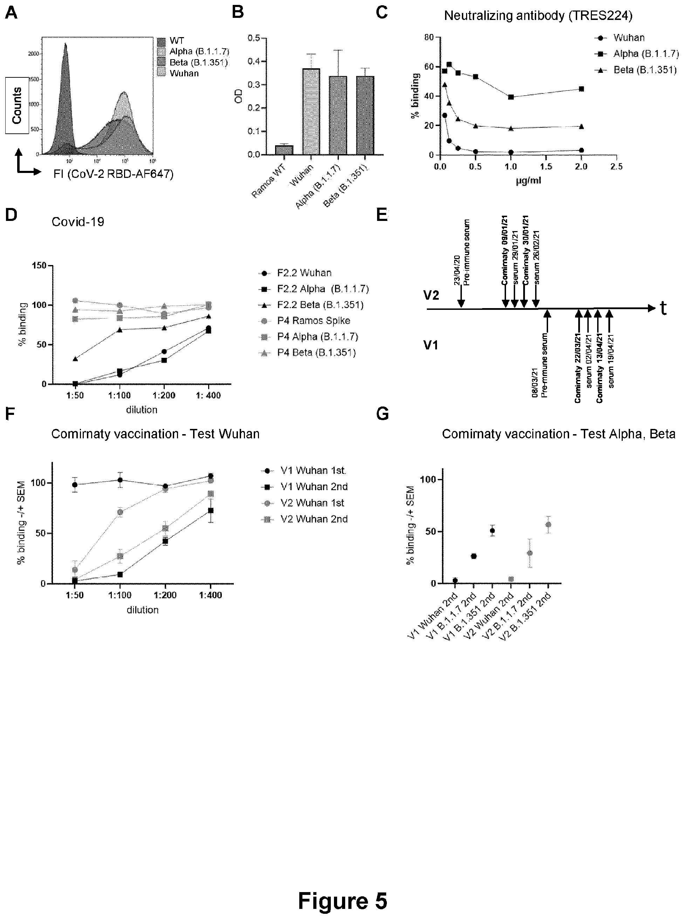

Figure 5 shows the application of SUBA in the testing of spike mutations and vaccination induced anti SARS-CoV-2 blocking activity
   **A** Flow cytometric analysis of Ramos cells expressing SARS-CoV-2 spike wildtype (Wuhan), α (B.1.1.7) or β (B.1.351) mutants with anti-RBD Ab TRES224 and secondary AF647-labelled anti-human IgG antibody. Representative data of three experiments. **B** SUBA assay for Ramos cells expressing SARS-CoV-2 spike wildtype (Wuhan, n=14), α (B.1.1.7, n=11) or β (B.1.351, n=11) mutants. Data are shown as mean -/+ SD from three experiments. **C.** Wuhan spike-expressing Ramos-null B cells or Ramos-null B cells expressing the α (B.1.1.7) or β (B.1.351) spike mutants (each 10⁵/well) were allowed to attach to hACE-2-coated wells in the presence of increasing concentrations of the monoclonal antibody TRES224 diluted with the assay medium. Data are presented as mean % binding relative to control (RSp cells in the absence of serum or blocking antibodies) performed in duplicates. **D** Wuhan spike-expressing Ramos-null B cells or Ramos-null B cells expressing the α (B.1.1.7) or β (B.1.351) spike mutants (each 10⁵/well) were allowed to attach to hACE-2-coated wells in the presence of decreasing dilutions of sera from COVID-19 positive (F2.2) or negative (P1) donors and SUBA was performed. Data are presented as mean % binding relative to control (cells in the absence of serum or blocking antibodies) performed in duplicates. Representative of two experiments. **E** Timeline for blood sampling before and after Comirnaty vaccination from two volunteers (V1 and V2). **F, G** Wuhan spike-expressing Ramos-null B cells or Ramos-null B cells expressing the α (B.1.1.7) or β (B.1.351) spike mutants (each 10⁵/well) were allowed to attach to hACE-2-coated wells in the presence of decreasing dilutions of sera from V1 and V2 donors and SUBA was performed. Data are presented as mean % binding -/+ SEM, relative to control (cells in the absence of serum or blocking antibodies) from two experiments performed in duplicates, with values from the pre-immune sera subtracted.

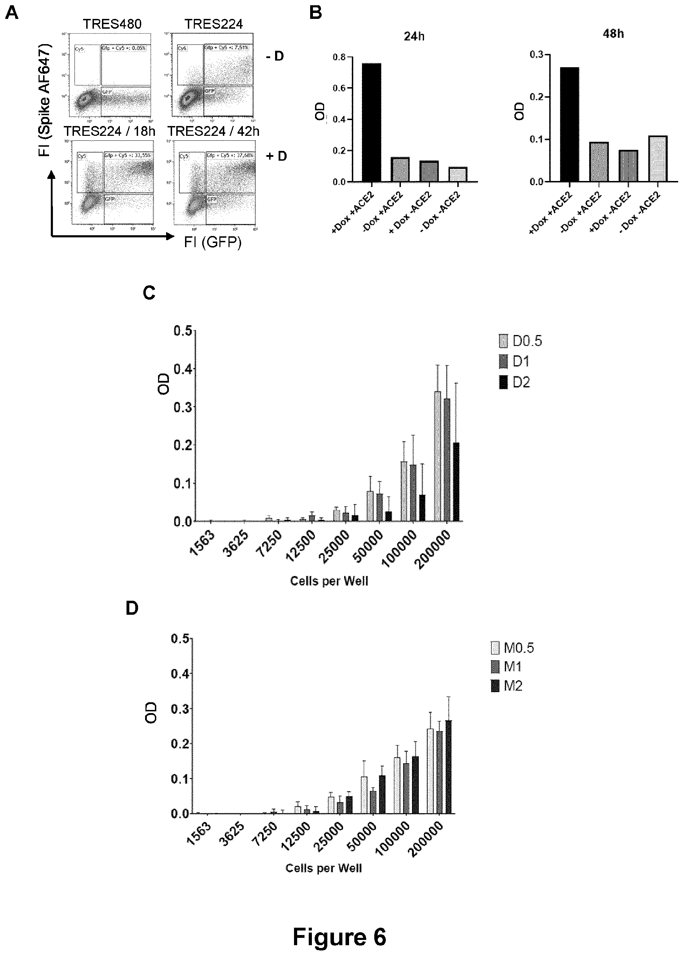

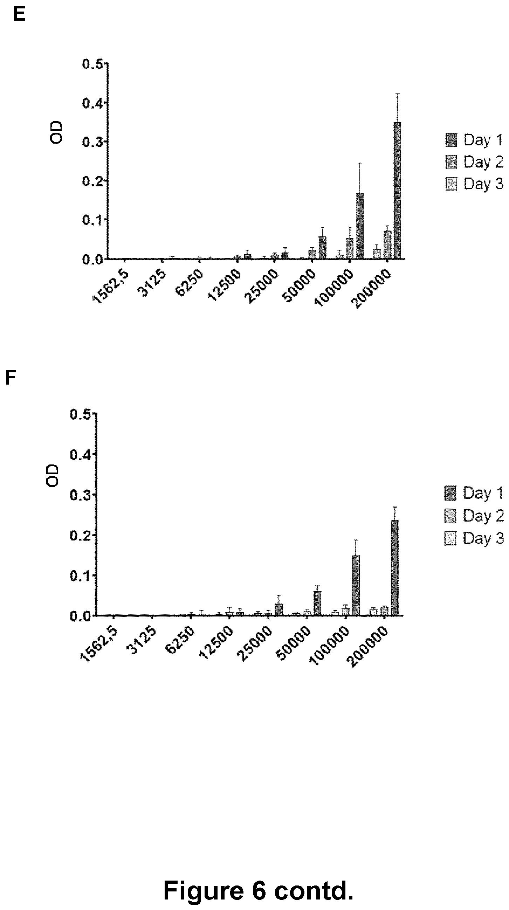

Figure 6 shows the Determination of parameters of the methods of the present invention for HEK293T cells.
   **A** HEK293T cells stably transfected with a Doxycycline-inducible SARS-CoV-2 spike protein (HEK-Dox-Spike) were grown in the absence or presence of Doxycycline (D, 1µg/ml) for 18 or 42 hours. Cells were stained with the recombinant spike-RBD-binding antibody TRES224, a neutralizing antibody, or an isotype-matched control antibody (TRES480) followed by secondary anti-human IgG antibody coupled to Alexa647 and analyzed by flow cytometry. **B** HEK-Dox-Spike cells were grown in the presence of Doxycycline (1µg/ml) for 24 and 48 hours. Increasing amounts of cells were allowed to attach to hACE-2- or non-coated plates, fixed and stained with crystal violet in the presence of sera from SARS-CoV-2 infected individuals. Cells were attached to hACE-2- or non-coated plates, fixed and stained with crystal violet. Crystal violet was solubilized and OD₅₇₀ₙₘ was measured. Representative data of 5 experiments is shown. **C** Determination of net HEK-Dox-Spike cell binding to hACE-2 with varying Doxycycline (D) concentrations (0.5-2µg/ml) with background (non-induced HEK-Dox-Spike cells) subtracted. **D** Determination of net HEK-Dox-Spike cell binding to hACE-2 with varying Minocycline (M) concentrations (0.5-2µg/ml), background (non-induced HEK-Dox-Spike cells) subtracted. **E** HEK-Dox-Spike cell binding to hACE-2 after 1, 2 or 3 days of Doxycycline (1µg/ml) application, background (non-induced HEK-Dox-Spike cells) subtracted. **F** HEK-Dox-Spike cell binding to hACE-2 after 1, 2 or 3 days of Minocycline (1µg/ml) application, background (non-induced HEK-Dox-Spike cells) subtracted.

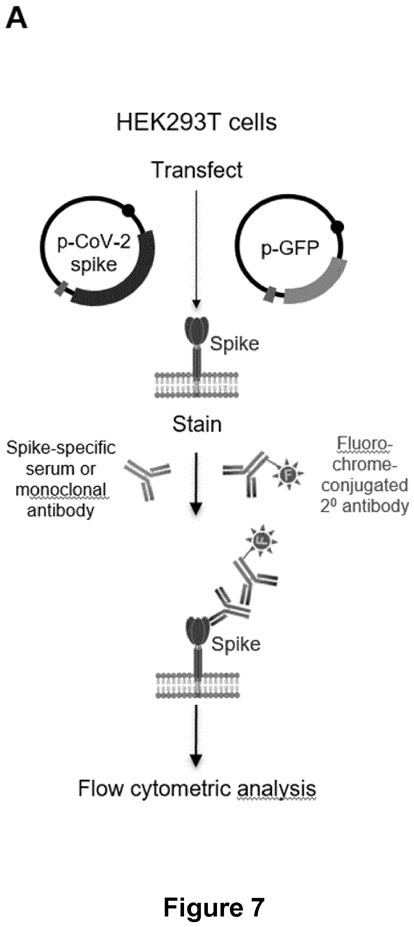

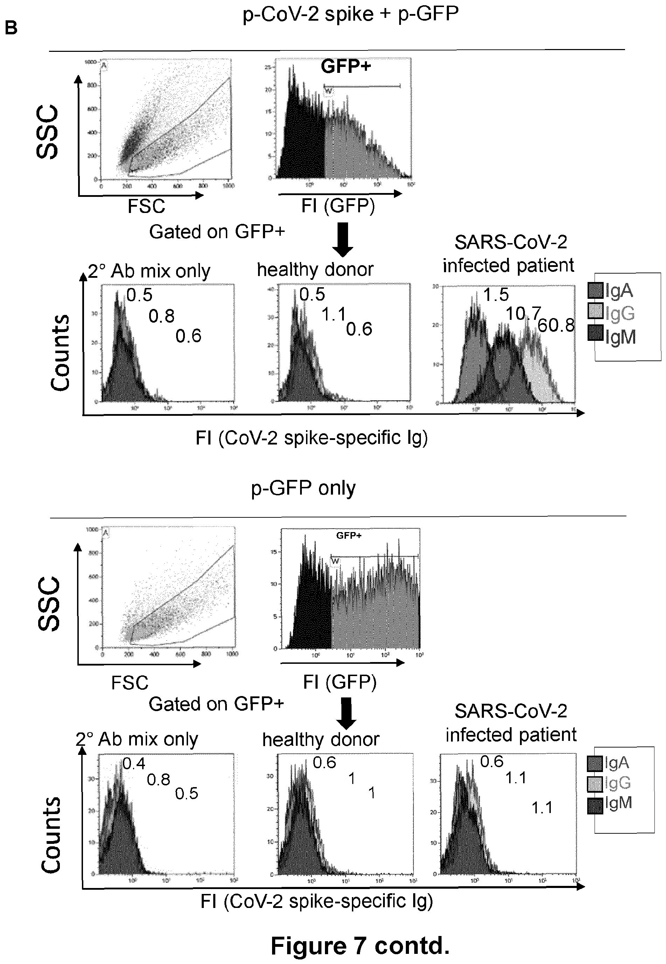

Figure 7 exhibits Flow cytometry-based analyses of SARS-Cov-2 spike-specific antibodies in the serum of patients.
   **A** Schematic overview: HEK293T cells were transiently transfected with a SARS-CoV-2 spike-encoding plasmid combined with a GFP-encoding plasmid. Two days after transfection, Spike-transfected HEK293T cells were stained with serum samples from infected patients and controls (1:100 dilutions) followed by staining with a secondary antibody mixture (2° antibody mix) consisting of PE-conjugated anti-human IgA, AF647-conjugated anti-human IgG and DyLight405-conjugated anti-human IgM antibody. **B** upper panel: Gating strategy for flow cytometric analysis: Living cells were gated based on FSC/SSC characteristics followed by gating on GFP⁺ cells. Histograms show fluorescence intensities for SARS-CoV-2 spike-specific IgM (red), IgA (blue) and IgG (green) serum antibodies. Numbers indicate mean fluorescence intensities (MFI). **B** lower panel: Flow cytometric control experiments for verification of spike-specificity using HEK293T cells only transfected with a GFP-encoding plasmid. GFP-transfected HEK293T cells were stained as described in **A, B** upper panel. Numbers indicate MFI.

### Details of the invention

The present invention provides means and methods for assaying a compound for its ability to block the binding of a viral surface protein to a human cell receptor. Preferably, the means and methods of the present invention are useful and/or used for assessing a compound for its ability to block the binding of the corona-spike protein, preferably SARS-CoV-2, to the human ACE-2 receptor. They can further be used for diagnosis of patients für a viral infection and for detecting the efficacy of therapeutics or potential therapeutics.

The term "corona virus spike protein" covers full-length corona virus spike proteins as known from the literature. The term, however, also includes functional fragments of the corona virus spike protein, preferably the S1 and the S2 fragment of corona virus spike protein of any of the known corona viruses and corona variants, such as the SARS-CoV-2 or its variants α, β, γ, δ, and ε. In a preferred embodiment, the corona virus spike protein is the receptor-binding domain (RBD) of the corona virus spike protein. Since the methods of the present invention seek to identify inhibitors of viral infections, preferably neutralizing inhibitors such as neutralizing antibodies, the corona virus spike protein preferably at least comprises and/or presents the amino acid sequences essential for the virus for cell entry, when the virus infects the cell. If the method of the present invention is used for assaying a compound for its ability to block the binding of a viral surface protein in embodiments envisaging viruses other than a coronavirus, another viral surface protein recognizing a cell receptor and effecting cell entry of the virus into the cell, shall be used instead of the corona virus spike protein. The term "viral surface protein" covers respective full length viral surface proteins as well as functional fragments capable of specifically binding to its respective cell receptor. A functional fragment preferably at least comprises and/or presents the amino acid sequence(s) essential the virus for cell entry.

The corona virus spike protein recognizes a receptor on cell surfaces, denominated "human ACE-2". Accordingly, the means and methods of the present invention refer to assaying for the blocking of the binding of the corona virus spike protein to human ACE-2 receptor. The term "human ACE-2 receptor" comprises the human ACE-2 as full receptor, its extracellular domain or any functional fragments thereof, capable of binding the spike protein, preferably the active domain of human ACE-2, i.e. the domain recognized by the coronavirus spike protein. Human ACE-2 preferably at least encompasses the amino acid sequence recognizing and/or binding to the corona virus spike protein, i.e. the corona virus spike protein's part essential for cell binding and/or cell entry. In embodiments wherein viruses other than the coronaviruses enter cells, human ACE-2 is replaced by other respective receptors known for the respective viruses. In the case of HIV, the viral surface protein (equivalent to the corona virus spike protein) is the viral surface protein gp120, or likewise gp41. The receptor (replacing the human ACE-2 receptor) is the CD4 receptor. The test, means and methods of the present invention are not limited to any particular virus surface protein, nor to its receptor counterpart on the cell surface, which together form the complex necessary for viral cell entry. The test, means and method of the present invention are useful for any virus, preferably any other pathogenic human virus with a known cell receptor used by the virus for cell entry. The "receptor" or "cell receptor" binds at least to the amino acid sequence of the viral surface protein, i.e. the viral cell protein's part essential for cell binding and/or cell entry. The cell receptor may be a full receptor, the extracellular domain thereof or a functional fragment thereof, preferably the active domain of cell receptor, i.e. the domain recognized by the viral surface protein.

In one aspect of the present invention, a method is provided of assaying a test compound for its ability to block the binding of the corona virus spike protein to the human ACE-2 receptor, the method comprising:
a) incubating human ACE-2 with:
   i) a ligand, which is a corona virus spike protein that maintains the ability to bind with specificity to human ACE-2; and
   ii) said test compound;
b) determining the amount of the ligand bound to said human ACE-2;
c) comparing the results obtained in step b) with those obtained for human ACE-2 incubated with the ligand but in the absence of said test compound; and
d) concluding that said test compound blocks the binding of the corona virus spike protein to human ACE-2, if the amount of binding observed in the presence of said test compound is lower than in the absence of said test compound.

Preferably, the viral surface protein, e.g. the corona virus spike protein, is in its native confirmation; "native confirmation" means, the viral surface protein , e.g. the corona virus spike protein, presents the same three-dimensional epitopes as presented on the (corona) virus capsid. The three-dimensional epitopes are preferably in the amino acid sequence necessary for cell receptor binding of the (corona) virus when cell entry of the (corna) virus is achieved.

In the present invention, the test compound is preferably an antibody, a small molecule or an anti-receptor protein. More preferably, the test compound is an antibody. The preferred goal of the present invention is to identify neutralizing inhibitors, more preferably neutralizing antibodies. The term "neutralizing antibody" means an antibody that prevents a cell viral infection. "Neutralization" or "neutralizing" means that an agent renders a virus no longer infectious and/or pathogenic.

Test compounds of the present invention comprise potentially therapeutic active substances. Such potentially therapeutic active substances may be derived from a serum of a patient, may be derived from a fungus or may be synthetic compounds, e.g. biomimetics. The test compounds may be a serum as taken from a patient or a compound in such a serum or maybe isolated from such a serum of a patient.

The methods of the present invention are useful to identify compounds or test compounds that are candidates for medicaments. The methods of the present invention may also be used to test medicaments before or after marketing authorization, particularly medicaments comprising a therapeutic antibody, preferably a neutralizing therapeutic antibody.

In accordance with the present invention the "human ACE-2 receptor" is a full cell receptor, its extracellular domain or a functional fragment of human ACE-2, which means a fragment that includes the amino acid sequence recognizing the corona virus spike protein necessary for the entry of the virus into the cell. In a preferred embodiment, the human ACE-2 is the full receptor, its extracellular domain or the active domain of human ACE-2.

According to the present invention, the human ACE-2 is preferably immobilized to a surface or is immobilized on a solid support.

A typical surfacescomprises a well plate, e.g. a 16-, 48-, 96- or 128- well plate, or any other analytical support used in the art for *in vitro* analysis. In one embodiment isolated membranes of cells expressing human ACE-2 on surfaces may be attached to the well plates.

In one preferred embodiment of the present invention, the corona virus spike protein (or the respective viral surface protein) is expressed on the surface of a cell. The expression on a cell has been identified by the present inventors to guarantee the presentation of the corona virus spike protein (or the respective viral surface protein) in its "native confirmation" as defined above. It surprisingly turned out that the tests of the present invention are superior to conventional surrogate virus neutralization tests.

In preferred embodiments, the cells expressing the corona virus spike protein (or the respective viral surface protein) o their surface are neither immobilized on a surface nor on a solid support. The preferred test format of the present invention is to provide the human ACE-2 or respective cell receptor immobilized on a surface and to have the cells with the expressed corona virus spike protein or viral surface protein in aqueous suspension. The cells in suspension may then contact the immobilized human ACE-2 (or respective cell receptor). Preferably, according to the present invention, the concentration of cells is 0.01 -1 x 10⁷ cells/ml aqueous medium, more preferably 0.5-4 x 10⁶ cells /ml aqueous medium. A typical assay volume is 10 - 1000 µl, preferably 100 µl per assay.

In another test format of the invention, the cells expressing the viral surface proteins (such as the corona virus spike protein) on their surface may be immobilized and the respective cell receptor (such as human ACE-2) is provided in suspension.

In the methods of the present invention, the corona virus spike protein is expressed on the surface of a cell at a density between 1-100 x 10⁴ corona virus spike protein molecules per cells. Preferred are 5-20 x 10⁴ corona virus spike protein molecules per cell.

The amount of corona virus spike protein per cell can also be determined as weight per cell. Preferred is an amount of between 0.25-25 x 10⁻¹⁴ g corona virus spike protein per cell, preferably 1.2-4.8 x 10⁻¹⁴ g corona virus spike protein per cell.

The viral surface protein according to the present invention may be any useful viral surface protein. If a corona virus spike protein is envisaged, then the corona virus spike protein can be any spike protein of a coronavirus, preferably a spike protein of SARS-CoV-2. Spike proteins of SARS-CoV-2 have been described in the literature and sequences of said spike proteins are available in the art. All of these sequences are envisaged. Further envisaged are variants of these sequences, in particular variants of existing spike proteins exhibiting 90%, 95%, 98%, 99%, 99.5%, 99.7%, 99.9% identity with any of the known corona virus spike proteins. Preferred sequences according to the present invention are SEQ ID NOs: 1-5 and the corona virus spike proteins of the present invention may either comprise any of SEQ ID Nos: 1-5 or a spike protein exhibiting 90%, 95%, 98%, 99%, 99.5%, 99.7% or 99.9% identity with any of SEQ ID NOs: 1-5. More preferred is a corona virus spike protein exhibiting at least 98%, identity with any of SEQ ID NOs. 1-5. Such variants cover alpha, beta, gamma and delta variants of SARS-CoV2 spike protein sequences. SEQ ID NO:1 encodes the full SARS-CoV-2 spike protein, SEQ ID NO:2 encodes the S1 sequence of the SARS-CoV-2 spike protein, SEQ ID NO:3 encodes the S2 sequence of the SARS-CoV-2 spike protein and SEQ ID NO:4 encodes the RBD sequence of the SARS-CoV-2 spike protein. Same applies to respective variants defined by %-identity. %-identity is determined with a conventional Computer algorithm established in the art.

In another embodiment, the corona virus spike protein of the invention comprises SEQ ID NO:5, which is the consensus full spike sequence shared by SARS-CoV-2, alpha, beta, gamma and delta variant. In still another embodiment, the corona virus spike protein of the invention comprises amino acids 13-685 of SEQ ID NO:5, which is the consensus S1 sequence shared by SARS-CoV-2 wt, alpha, beta, gamma and delta variant. In another embodiment, the corona virus spike protein of the invention comprises amino acids 685-1273 of SEQ ID NO:5, which is the consensus S2 sequence shared by SARS-CoV-2 wt, alpha, beta, gamma and delta variant. In still another embodiment, the corona virus spike protein of the invention comprises amino acids 301-550 of SEQ ID NO:5, which is the consensus RBD sequence shared by SARS-CoV-2 wt, alpha, beta, gamma and delta variant. Using one of these sequences as defined above by reference to SEQ ID NO:5 or its above defined S1, S2 and RBD fragments, the tests, cells and methods of the present invention are capable of recognizing all SARS-CoV-2 variants with optimal accuracy. The test, cells and methods are thus useful for the identification of universal compounds neutralizing all SARS-CoV-2 variants.

Furthermore preferred are the corona virus spike proteins from one of the seven known human corona virus species, which are classified into two genera: Alpha-coronaviruses (HCoV-229E, HCoV-NL63) and Beta-coronaviruses (HCoV-HKU1, HCoV-OC43, SARS-CoV, MERS-CoV, SARS-CoV-2).

To identify test compounds capable of inhibiting, preferably neutralizing, corona virus mutants, the corona virus spike protein may be from a SARS-CoV-2 variant, preferably a variant occurring due to mutation, e.g. α, β, δ, ε variant of SARS-CoV-2.

It is known in the art that the human ACE-2 receptor is the same for all SARS coronaviruses. Other coronaviruses apart from SARS can have other cell receptors, which can then be used according to the means and method of the present invention, e.g. as "purified cell receptors".

Percent identity values are determined with conventional algorithms and computer software established in the art.

Cells expressing the viral surface protein, preferably the corona virus spike protein on their surface are preferably prepared by transfection. Accordingly, the method of the present invention comprise cells expressing the viral surface protein, preferably the corona virus spike protein on their surface, which are cells that have been transfected with a vector comprising a promoter operably linked to a sequence coding for the corona virus spike protein. The cells may be prepared by any recombinant technique known in the art to incorporate the corona virus spike protein, for example by transfection of adherent cells or of cells in suspension. Also envisaged is the ecotropic retroviral transduction or lentiviral transduction by standard recombinant techniques. In the current format, ecotropic retroviral transduction of human Ramos B cells is achieved by ectopic rexpression of an ecotropic retroviral receptor in Ramos B cells.

According to the methods of the present invention, the cells expressing the corona virus spike virus are in one embodiment detectably labelled, i.e. a "detectable label" or "label" Is attached. "Detectable label" or "label" are synonyms, herein.

Preferably, the detectable label is introduced into the cells before or after the binding of human ACE-2 (or the cell receptor) to the corona virus spike protein (or the viral surface protein). In other embodiments, the label may be present in the cell, e.g. as a native enzyme such as hexosamidase. The cell may also be a cell naturally encompassing or recombinantly modified to express a fluorescent label such as the green fluorescent protein (GFP) or a derivative thereof such as BFP, et cetera. The cell may also be a cell that has been modified to incorporate a label such as GFP or BFP or any other dye molecule or a fluorophore known in the art. These labels may e.g. be incorporated by transfection or infection via a recombinant technique before or after binding of human ACE-2.

In another embodiment, the detectable label may be incorporated into the cells, or may be attached to the cell surface. For example, the cell DNA or cell proteins may be stained by the label. In a preferred embodiment of the present invention the detectable label comprises or is crystal violet. In another preferred embodiment the detectable label comprises, or is propidium iodide. Another detectable label comprises or is Coomassie blue or any commercial fluorophore or dye molecule.

In another embodiment, the detectable label may be attached to the test compound after binding between the viral surface protein and its receptor; e.g. by a secondary antibody against a test compound which itself may be an antibody or the label may be attached to the test compound by using the binding pair biotin and streptavidin.

The detectable labelling of the present invention is not particularly limited. While it is preferred that the detectable label is used to identify the complex of cell receptor and-viral surface protein (e.g. the human ACE-2 - corona virus spike - complex), it is also possible that the detectable label is attached to unbound cell receptor (e.g. unbound human ACE-2), or the unbound viral surface protein (unbound spike protein), e.g. the human ACE-2 (or other cell receptor) and the viral surface protein (corona virus spike) not attached to each other are detectably labelled, whereas the complex is not.

The detectable labelling and/or the methods of the present invention may also make use of a competitive reaction as established in the art.

The type of detectable label is not particularly limited. Whereas dye molecules, fluorophores, or fluorochromes are preferred, also radio-labelling techniques are envisaged. Most preferred is crystal violet.

The label may stain proteins, nucleic acids, or carbohydrates of the cell.

The detectable label may be incorporated into the cells expressing the viral surface protein, e.g. the corona virus spike protein. The incorporation of the detectable label can be achieved by staining cells with labels after fixation and/or permeabilization of cells or by transfection or infection of cells with a detectable label, for example a fluorescent protein produced by recombinant techniques.

The methods of the present invention preferably comprise a step of detecting a signal intensity. The signal intensity is preferably a fluorescent signal or an absorption or reflection signal, which correlates with the amount of detectable label, which is preferably attached to the cell bearing the viral surface protein, such as the corona virus spike protein, on its surface, wherein the viral surface protein is bound to the respective receptor, such as human ACE-2. Preferably, the detection encompasses the detection of an optical signal, albeit a radiolabel signal can also be detected. The step of signal detection preferably takes place in an aqueous medium. In one embodiment, the cells are lysed to release the label into a medium, which is preferably an aqueous medium, before signal detection. For the detection of the signal, preferably a photometer is used. The optical signal may be detected and quantified, for example the optical density OD is detected and quantified.

One means which can be used in the methods of the present invention is a "cell" or "host cell" - both terms being used as synomyms, herein - which expresses a viral surface protein, preferably a corona virus spike protein on its surface. Such a cell can be transferred to the customers or can be used in a diagnostic laboratory.

Preferably, the cell presents and/or provides the viral surface protein, in a preferred embodiment the corona virus spike protein, in its native confirmation. In one aspect, the cell is a mammalian cell, a yeast cell or a single cell algae. Preferred cells are Ramos cells, or HEK 293 cells.

The viral surface protein expressed on the cell surface is any viral surface protein useful according to the present invention. Preferably, the cell expresses on its surface a corona virus spike protein as defined herein and used in any of the methods of the present invention.

The cells according to the present invention and useful for the method of the present invention may be prepared by a recombinant technique. They may for example be prepared by a recombinant technique useful for incorporating a viral surface protein into the cell, such as the corona virus spike protein, e.g. by transfection of adherent cells or in suspension, by ecotropic retroviral transduction or by lentiviral transduction

In one aspect of the present invention, a method is provided for preparing the cells of the present invention. Such a method comprises the following steps:
a) preparing a construct encoding the viral surface protein, e.g. corona virus spike protein, preferably by a recombinant technique,
b) purifying the construct,
c) transfecting the cells by using liposomes or calcium phosphate,
d) selecting transfected cells, e.g. by antibiotic resistance, and
e) optionally performing a quality control by flow cytometric detection of the viral surface protein, e.g. the corona virus spike protein.

The cells prepared are also provided by the present invention.

In a further embodiment a method is provided for preparing the cells of the present invention comprising transduction. Such a method comprises the steps of:
a) preparing a construct encoding the viral surface protein, e.g. corona virus spike protein, preferably by a recombinant technique,
b) purifying the construct,
c) transfecting packaging cells by liposomes or calcium phosphate, wherein the packaging cells are preferably Phoenix Eco,
d) collecting the supernatant of the packaging cells,
e) infecting of the cells with the supernatant.
f) selecting the infected cells, e.g. by antibiotics, and/or purification to homogeneity by cell sorting, and
g) optionally performing quality control by flow cytometric detection of the virus surface protein, e.g. the corona virus spike protein.

The cells prepared also provided by the present invention. In this embodiment, the cells are preferably Ramos cells.

The cells of the present invention can be conserved and/or stored. For example they can be conserved by freezing in a medium at -70°C. For long-term storage temperature of -180°C is useful.

The cells of the invention may be transferred to the customers, e.g. in a freeze-dried stage. The customers may revive and/or cultivate cells before using them in one of the methods of the present invention. Cell cultivation can be performed using standard conditions. Cell revival can be performed under standard conditions.

The cells of the invention may also be transferred, e.g. shipped, to customers upon mild fixation in that form, which still allows the surrogate binding assay, in particular if the staining of the cells has been performed before.

The cells of the invention may be detectably labelled by any of the labelling techniques and/or labels as described throughout the present specification.

Provided is further the use of any of the cells of the present invention in a surrogate corona virus neutralization test or assay. Also envisaged is the use of any of the host cells of the present invention in a surrogate virus neutralization test for any other virus known in the art, e.g. HIV. The viral surface protein, e.g. the corona virus spike protein and the receptor, such as the human ACE-2 receptor, are as defined throughout the present specification.

Further provided is a transfection vector comprising a nucleic acid sequence encoding an amino acid of a viral surface protein, such as an amino acid sequence of a corona virus spike protein.

The transfection vector of the present invention may further encode an internal ribosomal entry site (IRES). The IRES may be coupled to a fluorescent protein and/or an enzyme, e.g. to GFP or BFP or hexosaminidase, beta galactosidase or others. Accordingly, the respective transfection vector encodes an amino acid sequence of IRES and encodes the respective amino acid sequence encoding a detectable label.

Further provided by the present invention is a method of assaying, i.e. a method of assaying a test compound for its ability to block the binding of a viral surface protein to a cell receptor, preferably a human cell receptor, comprising:
a) Incubating the cell receptor with:
   i) a ligand, which is a viral surface protein that maintains the ability to bind with specificity to the cell receptor; and
   ii) said test compound;
b) determining the amount of the ligand bound to said cell receptor;
c) comparing the results obtained in step b) with those obtained for a cell receptor incubated with the ligand but in the absence of said test compound; and
d) concluding that said test compound blocks the binding of the viral surface protein to the cell receptor, if the amount of binding observed in the presence of said test compound is lower than in the absence of said test compound.

Preferably, the viral surface protein is in its native conformation.

Further embodiment are:
- The virus is HIV and the viral surface protein is gp120
- The virus is a SARS-Coronavirus (SARS-CoV-1) or a MERS virus and the viral surface protein is the spike of SARS-CoV-1 or a MERS virus
- The virus is any other pathogenic human virus with a known receptor
- The virus is a SARS- CoV-2, which is preferred.
- The virus may be a virus infecting a mammal or another species apart from human patients, viruses infecting human patients are of preferred interest.

All preferred embodiments of the invention defined for a SARS-CoV-2 (with corona virus spike protein and human ACE-2) apply *mutatis mutandis* to the above method for any other viruses (with respective viral surface proteins and cell receptors).

Further provided is a method of analyzing a sample as follows:
A method of analysing a sample for the presence of a compound capable of neutralizing corona virus infection, comprising:
- contacting the sample with the corona virus spike protein, and the human ACE-2 protein, and
- determining the level of interaction between the corona virus spike protein and the human ACE-2.

A reduced level of interaction between the corona virus spike protein and the human ACE-2 indicates the presence of a compound capable of at least party inhibiting the interaction. The corona virus spike protein and the human ACE-2 may be as defined in the present specification.

In this embodiment the sample may be a blood sample, a lymph sample, a saliva sample, or a synovial fluid sample or the sample may comprise a test compound. The test compound may be any test compound as described in this specification.

Further provided is a method for diagnosis as follows:
A method for diagnosis of a corona virus infection, comprising
- providing a sample previously collected from the subject to be diagnosed,
- contacting the sample with a spike protein as cited in any of items 1-11 and an ACE-2 protein as cited in any of items 1-11;
- determining the level of interaction between the spike protein and the ACE-2;
wherein the reduction of the interaction level in the presence of the sample indicates the subject is infected or was infected with a SARS-CoV.

Preferably, the coronavirus is SARS-CoV-2 or a variant thereof. The corona virus spike protein and the human ACE-2 may be as defined in the present specification.

Furthermore, provided is a kit comprising a cell of the present invention.

In a preferred embodiment, the kit comprises in addition to said cell a recombinant cell receptor protein, for example human ACE-2, more preferably a purified cell receptor protein.

Further preferred is a kit comprising the cell of the present invention together with a detectable label, preferably the detectable label is contained in the cells. In a further preferred embodiment, the kit comprises the cell, the recombinant receptor protein, e.g. human ACE-2, and a detectable label, wherein preferably the label is contained in the cell, and most preferably wherein the label is crystal violet.

### Examples

### 1. Antibodies

The anti-SARS-CoV-2 RBD antibody TRES224 (human IgGκ) (34) has been described previously and was produced by Celltheon (San Francisco, CA). TRES328 (human IgG1κ, NTD binder), TRES480 and TRES567 (human IgG1κ, non-binders) were generated during the same immunization and purified from transfected HEK293T cells according to standard procedures (Peter et al., manuscript in preparation). For flow cytometric analyses, anti-human IgG FITC antibody and DyLight405-conjugated anti-human IgM were purchased from Jackson (Dianova, Hamburg), PE-conjugated anti-human IgA and AF647-conjugated anti-human IgG were obtained from Southern Biotech (Birmingham, AL). Anti-SARS-CoV-2 RBD antibody R10933 (Regeneron) (19) was produced by Sino Biological (Eschborn, Germany).

### 2. Plasmids

To generate an inducible vector for SARS-CoV-2 spike protein expression, the open reading frame of SARS-CoV-2 spike was amplified from pCG1_CoV_2019S (encoding the Wuhan-Hu-1 Spike protein (QHD43416.1, AA1-1273, position 21580-25400 from Genbank NC_045512)) by polymerase chain reaction (PCR) with the primers fw_Nhel (5'-ctggctagcgccaccatgtttctgctgaccacc-3') and rev_Stul (5'-tcaaggcctttaggtgtagtgcagtttcacgccc-3') for 30 cycles at 65°C annealing temperature. The PCR product was purified, digested with *Nhe*I and *Stu*I and cloned into *Nhe*I and EcoRV digested pWHE469 (33), followed by sequencing.

### 3. Cell lines

Ecotropic Ramos-null cells have been described (32) and were cultured in fully supplemented RPMI1640 medium (Gibco) containing 10% fetal calf serum (FCS) at 5% CO₂ in a fully humidified incubator at 37°C. Ramos-null cells were infected with retroviral particles derived from pMIG (https://www.addgene.org/9044/) encoding the Wuhan-Hu-1 Spike protein (QHD43416.1, AA1-1273, position 21580-25400 from Genbank NC_045512) and then sorted for GFP expression to obtain Ramos-null spike (RSp) cells. HEK293T cells were cultured in fully supplemented DMEM medium (Gibco) containing 10% fetal calf serum (FCS) at 7.5% CO₂ in a fully humidified incubator at 37°C. HEK293T cells were transfected with the linearized plasmid pWHE469-SARS-CoV-2 spike by Lipofectamine (Gibco) and selected with Puromycin (Gibco, 2µg/ml).

### 4. Recombinant proteins

The plasmid encoding the ectodomain of human ACE-2 (hACE-2) (AA18-738, NP_001358344.1) with an IgLκ signal peptide fused to a human IgG1 Fc part followed by Myc and His-Tag was cloned in pCEP4 (Thermo Fisher). Recombinant hACE-2 was produced with the FreeStyle 293 Expression System (Fisher Scientific) by transfecting FreeStyle 293-F cells kept in Freestyle 293 Expression Medium with the pCEP4-hACE-2 plasmid. hACE-2 was affinity-purified from filtered cultured supernatant on a High-Trap Protein G column (GE Healthcare, Chicago, USA), immediately neutralized, dialyzed against phosphate buffered saline and stored in 50% glycerol at -20°C. The quality of protein purity was assessed by SDS-PAGE Coomassie staining. Protein concentrations were determined by OD at 280nm and verified by a Bradford assay (Thermo Fisher Scientific, Waltham, USA).

### 5. Serum samples

Serum samples were analyzed in the context of a longitudinal study of 56 households with at least one member who had COVID-19 to investigate the course of illness, immune responses, and long-term consequences of SARS-CoV-2 infection in patients with ectodermal dysplasia and control subjects of the same age group (ClinicalTrials.gov identifier: NCT04741412). This study was approved by the ethics committee of the University Erlangen-Nürnberg and conducted in accordance with the principles of the declaration of Helsinki. All individuals provided written informed consent to participate.

### 6. Cell-based surrogate SARS-CoV-2 blocking assay (SUBA)

Flat bottom ELISA plates were coated with 50µl/well of 20 µg/ ml hACE-2 in coating buffer (15 mM Na₂CO₃, 35 mM NaHCO₃) overnight at 4°C. Plates were washed three times with phosphate buffered saline (PBS) and blocked with 100 µl of 10 % bovine serum albumin (BSA) in PBS for one hour at room temperature. Plates were washed twice with PBS and 10⁵ cells in 100 µl full medium containing the desired serum or antibody were plated per well (complement inactivation of human serum for 30 min at 56°C may be necessary if serum containing B cell depleting antibodies is analyzed). Plates were incubated for 1 h at 37°C in an incubator and then emptied into the sink with momentum. Plates were then placed on ice and wash twice with 200 µl/ well of cold PBS by gently tapping the plate on paper towels. Cells were then fixed cells on ice for 10 min with 200 µl/ well of ice-cold pure methanol, methanol was emptied with vigor and plates were tapped twice gently on paper towel. The plate was then stained at room temperature for 10 min with 50 µl/ well of 0.5% crystal violet solution in 25% methanol and washed three times with de-ionized water in a large container by submersion. The plate was gently tapped twice on a paper towel and 100 µl/well of a 1% sodium dodecyl sulfate solution were added. (http://www2.kumc.edu/soaiab/LabLinks/protocois/cvassay.htm). Using a multichannel pipette, the fixed crystal violet was dissolved to homogeneity and plates were analyzed in an ELISA reader at 570nm.

### 7. Flow cytometry-based analyses of SARS-CoV-2 spike-specific antibodies in the serum of patients.

HEK293T cells were transiently transfected using the PEI method with the SARS-CoV-2-spike-encoding plasmid pCG1_CoV_2019S (38) in combination with a GFP encoding plasmid. HEK293T cells transfected only with a GFP-encoding plasmid served as a negative control. Two days after transfection, SARS-CoV-2 spike/GFP-co-transfected and GFP-only transfected HEK293T cells were stained with serum samples (1:100 dilution) from SARS-CoV-2-infected patients and non-infected controls followed by staining with a secondary antibody mixture (2° antibody mix) consisting of PE-conjugated anti-human IgA (Southern Biotech, Birmingham, AL), AF647-conjugated anti-human IgG (Southern Biotech, Birmingham, AL) and DyLight405-conjugated anti-human IgM (Jackson Immuno Research, Dianova, Hamburg) antibodies. Cells were analyzed using a Gallios flow cytometer (Beckman Coulter).

### References:

1. Hu B, Guo H, Zhou P, and Shi Z-L. Characteristics of SARS-CoV-2 and COVID-19. Nature Reviews Microbiology. 2021;19(3):141-54.
2. Pezzini A, and Padovani A. Lifting the mask on neurological manifestations of COVID-19. Nat Rev Neurol. 2020;16(11):636-44.
3. Yang X, Yu Y, Xu J, Shu H, Xia Ja, Liu H, et al. Clinical course and outcomes of critically ill patients with SARS-CoV-2 pneumonia in Wuhan, China: a single-centered, retrospective, observational study. Lancet Respir Med. 2020;8(5):475-81.
4. Wajnberg A, Amanat F, Firpo A, Altman DR, Bailey MJ, Mansour M, et al. Robust neutralizing antibodies to SARS-CoV-2 infection persist for months. Science (New York, N Y). 2020;370(6521):1227-30.
5. To KK-W, Tsang OT-Y, Leung W-S, Tam AR, Wu T-C, Lung DC, et al. Temporal profiles of viral load in posterior oropharyngeal saliva samples and serum antibody responses during infection by SARS-CoV-2: an observational cohort study. Lancet Infect Dis. 2020;20(5):565-74.
6. Long Q-X, Liu B-Z, Deng H-J, Wu G-C, Deng K, Chen Y-K, et al. Antibody responses to SARS-CoV-2 in patients with COVID-19. Nat Med. 2020;26(6):845-8.
7. Brouwer PJM, Caniels TG, van der Straten K, Snitselaar JL, Aldon Y, Bangaru S, et al. Potent neutralizing antibodies from COVID-19 patients define multiple targets of vulnerability. Science (New York, N Y). 2020;369(6504):643-50.
8. Robbiani DF, Gaebler C, Muecksch F, Lorenzi JCC, Wang Z, Cho A, et al. Convergent antibody responses to SARS-CoV-2 in convalescent individuals. Nature. 2020;584(7821):437-42.
9. Zost SJ, Gilchuk P, Case JB, Binshtein E, Chen RE, Nkolola JP, et al. Potently neutralizing and protective human antibodies against SARS-CoV-2. Nature. 2020;584(7821):443-9.
10. Sahin U, Muik A, Derhovanessian E, Vogler I, Kranz LM, Vormehr M, et al. COVID-19 vaccine BNT162b1 elicits human antibody and TH1 T cell responses. Nature. 2020; 586(7830): 594-9.
11. Anderson EJ, Rouphael NG, Widge AT, Jackson LA, Roberts PC, Makhene M, et al. Safety and Immunogenicity of SARS-CoV-2 mRNA-1273 Vaccine in Older Adults. N Engl J Med. 2020;383(25):2427-38.
12. Gaebler C, Wang Z, Lorenzi JCC, Muecksch F, Finkin S, Tokuyama M, et al. Evolution of Antibody Immunity to SARS-CoV-2. bioRxiv: the preprint server for biology. 2020.
13. Widge AT, Rouphael NG, Jackson LA, Anderson EJ, Roberts PC, Makhene M, et al. Durability of Responses after SARS-CoV-2 mRNA-1273 Vaccination. N Engl J Med. 2021;384(1):80-2.
14. Barnes CO, West AP, Jr., Huey-Tubman KE, Hoffmann MAG, Sharaf NG, Hoffman PR, et al. Structures of Human Antibodies Bound to SARS-CoV-2 Spike Reveal Common Epitopes and Recurrent Features of Antibodies. Cell. 2020;182(4):828-42 e16.
15. Dan JM, Mateus J, Kato Y, Hastie KM, Yu ED, Faliti CE, et al. Immunological memory to SARS-CoV-2 assessed for up to 8 months after infection. Science (New York, NY). 2021;371(6529).
16. Hartley GE, Edwards ESJ, Aui PM, Varese N, Stojanovic S, McMahon J, et al. Rapid generation of durable B cell memory to SARS-CoV-2 spike and nucleocapsid proteins in COVID-19 and convalescence. Sci Immunol. 2020;5(54).
17. Rodda LB, Netland J, Shehata L, Pruner KB, Morawski PA, Thouvenel CD, et al. Functional SARS-CoV-2-Specific Immune Memory Persists after Mild COVID-19. Cell. 2021;184(1):169-83 e17.
18. Cao Y, Su B, Guo X, Sun W, Deng Y, Bao L, et al. Potent Neutralizing Antibodies against SARS-CoV-2 Identified by High-Throughput Single-Cell Sequencing of Convalescent Patients' B Cells. Cell. 2020; 182(1):73-84 e16.
19. Hansen J, Baum A, Pascal KE, Russo V, Giordano S, Wloga E, et al. Studies in humanized mice and convalescent humans yield a SARS-CoV-2 antibody cocktail. Science (New York, NY). 2020;369(6506):1010-4.
20. Walls AC, Park Y-J, Tortorici MA, Wall A, McGuire AT, and Veesler D. Structure, Function, and Antigenicity of the SARS-CoV-2 Spike Glycoprotein. Cell. 2020;181(2):281-92.e6.
21. Zhou D, Duyvesteyn HME, Chen CP, Huang CG, Chen TH, Shih SR, et al. Structural basis for the neutralization of SARS-CoV-2 by an antibody from a convalescent patient. Nat Struct Mol Biol. 2020;27(10):950-8.
22. Focosi D, Maggi F, Mazzetti P, and Pistello M. Viral infection neutralization tests: A focus on severe acute respiratory syndrome-coronavirus-2 with implications for convalescent plasma therapy. Rev Med Virol. 2020:e2170.
23. Lau EHY, Tsang OTY, Hui DSC, Kwan MYW, Chan WH, Chiu SS, et al. Neutralizing antibody titres in SARS-CoV-2 infections. Nature communications. 2021;12(1):63.
24. Schmidt F, Weisblum Y, Muecksch F, Hoffmann HH, Michailidis E, Lorenzi JCC, et al. Measuring SARS-CoV-2 neutralizing antibody activity using pseudotyped and chimeric viruses. J Exp Med. 2020;217(11).
25. Toon K, Bentley EM, and Mattiuzzo G. More Than Just Gene Therapy Vectors: Lentiviral Vector Pseudotypes for Serological Investigation. Viruses. 2021;13(2).
26. Byrnes JR, Zhou XX, Lui I, Elledge SK, Glasgow JE, Lim SA, et al. Competitive SARS-CoV-2 Serology Reveals Most Antibodies Targeting the Spike Receptor-Binding Domain Compete for ACE-2 Binding. mSphere. 2020;5(5).
27. Walker SN, Chokkalingam N, Reuschel EL, Purwar M, Xu Z, Gary EN, et al. SARS-CoV-2 Assays To Detect Functional Antibody Responses That Block ACE-2 Recognition in Vaccinated Animals and Infected Patients. J Clin Microbiol. 2020;58(11).
28. Kemp SA, Collier DA, Datir RP, Ferreira I, Gayed S, Jahun A, et al. SARS-CoV-2 evolution during treatment of chronic infection. Nature. 2021.
29. Oladunni FS, Park JG, Chiem K, Ye C, Pipenbrink M, Walter MR, et al. Selection, identification, and characterization of SARS-CoV-2 monoclonal antibody resistant mutants. Journal of virological methods. 2021;290:114084.
30. Koenig PA, Das H, Liu H, Kümmerer BM, Gohr FN, Jenster LM, et al. Structure-guided multivalent nanobodies block SARS-CoV-2 infection and suppress mutational escape. Science (New York, NY). 2021;371(6530).
31. Klein S, Cortese M, Winter SL, Wachsmuth-Melm M, Neufeldt CJ, Cerikan B, et al. SARS-CoV-2 structure and replication characterized by in situ cryo-electron tomography. Nature communications. 2020;11(1):5885.
32. He X, Klasener K, lype JM, Becker M, Maity PC, Cavallari M, et al. Continuous signaling of CD79b and CD19 is required for the fitness of Burkitt lymphoma B cells. EMBO J. 2018;37(11).
33. Krueger C, Danke C, Pfleiderer K, Schuh W, Jack HM, Lochner S, et al. A gene regulation system with four distinct expression levels. J Gene Med. 2006;8(8):1037-47.
34. Pfister F, Vonbrunn E, Ries T, Jack HM, Uberla K, Lochnit G, et al. Complement Activation in Kidneys of Patients With COVID-19. Front Immunol. 2020;11:594849.
35. Sterlin D, Mathian A, Miyara M, Mohr A, Anna F, Claer L, et al. IgA dominates the early neutralizing antibody response to SARS-CoV-2. Sci Transl Med. 2021;13(577).
36. Garcia-Beltran WF, Lam EC, Astudillo MG, Yang D, MillerTE, Feldman J, et al. COVID-19-neutralizing antibodies predict disease severity and survival. Cell. 2021;184(2):476-88 e11.
37. Seow J, Graham C, Merrick B, Acors S, Pickering S, Steel KJA, et al. Longitudinal observation and decline of neutralizing antibody responses in the three months following SARS-CoV-2 infection in humans. Nat Microbiol. 2020;5(12):1598-607.
38. Lapuente D, Maier C, Irrgang P, Hubner J, Peter AS, Hoffmann M, et al. Rapid response flow cytometric assay for the detection of antibody responses to SARS-CoV-2. Eur J Clin Microbiol Infect Dis. 2020.
39. Valcourt et al Evaluation of a commercially-available surrogate virus neutralization test for severe acute respiratory syndrome coronavirus-2 (SARS-CoV-2). Diagnostic Microbiology and Infectious Disease Volume 99, Issue 4, April 2021, 115294
40. Tan et al, A SARS-CoV-2 surrogate virus neutralization test based on antibody-mediated blockage of ACE-2-spike protein-protein interaction, Nature Biotechnology volume 38, pages 1073-1078 (2020)

## Claims

1. A method of assaying a compound for its ability to block the binding of the corona virus spike protein to the human ACE-2 receptor, comprising:
a) incubating human ACE-2 with:
i) a ligand, which is a corona virus spike protein or a functional derivative thereof that has the ability to specifically bind with human ACE-2; and
ii) said compound;
b) determining the amount of the ligand bound to said human ACE-2;
c) comparing the results obtained in step b) with those obtained for human ACE-2 incubated with the ligand but in the absence of said compound; and
d) concluding that said compound blocks the binding of the corona virus spike protein to human ACE-2, if the amount of binding observed in the presence of said compound is lower than in the absence of said compound.

2. The method of claim 1, wherein the human ACE-2 is immobilised on a solid support.

3. The method of any of claims 1-2, wherein the corona virus spike protein or functional derivative thereof is expressed on the surface of a cell, wherein the cells are suspended in an aqueous suspension.

4. The method of any of claims 1-3, wherein the corona virus spike protein or functional derivative thereof is expressed on the surface of a cell in a density of between 1 -100 x 10⁴ corona virus spike protein molecules per cell.

5. The method of any of claims 1-4, wherein the corona virus spike protein or functional derivative thereof is selected from
a) the spike protein of SARS-CoV2 or a functional derivative thereof,
b) a variant thereof, such as alpha, beta, delta variant of SARS-CoV-2, or functional derivative thereof, and
c) a receptor binding domain (RBD) of the spike protein of a) or b).

6. The method of any of claims 1-5, wherein
a) the corona virus spike protein or functional derivative thereof comprises an amino acid sequence selected from any of SEQ ID NOs: 1-5, or
b) the corona virus spike protein or functional derivative thereof comprises an amino acid sequence that is at least 95% identical to any of SEQ ID NOs: 1-5.

7. The method of any of claims 4-6, wherein said cells expressing the corona virus spike protein are detectably labelled.

8. The method of claim 7, wherein the cells are detectably labelled with a label that is crystal violet or green fluorescent protein.

9. The method of any of claims 7-8, wherein the label is contained in the cells expressing the corona virus spike protein and the method further comprises the steps of
lysing the cells to release the label into an aqueous medium and
detecting an optical signal intensity of the medium comprising the released label.

10. A host cell expressing a corona virus spike protein or functional derivative thereof on its surface, optionally further including a detectable label.

11. Use of the host cell of claim 10 in a surrogate coronavirus neutralization test.

12. A transfection vector comprising a nucleic acid sequence encoding an amino acid sequence of a corona virus spike protein or functional derivative thereof, as defined in any of claims 1-6.

13. A method of assaying a compound for its ability to block the binding of a viral surface protein to a cell receptor, comprising:
a) incubating the cell receptor with:
i) a ligand, which is a viral surface protein that has the ability to bind with specificity to the cell receptor; and
ii) said compound;
b) determining the amount of the ligand bound to said cell receptor;
c) comparing the results obtained in step b) with those obtained for a cell receptor incubated with the ligand but in the absence of said compound; and
d) concluding that said compound inhibits the binding of the viral surface protein to the cell receptor, if the amount of binding observed in the presence of said compound is lower than in the absence of said compound.

14. A method of analyzing a sample for the presence of a compound capable of inhibiting and/or neutralizing a corona virus infection, comprising:
a) contacting the sample with the corona virus spike protein as cited in any of claims 1-11, and the ACE-2 protein as cited in any of claims 1-11, and
b) determining the level of interaction between the corona virus spike protein and the ACE-2, wherein a reduced interaction indicates the presence of an inhibiting compound.

15. A method for diagnosis of an infection with a corona virus, comprising
a) providing a sample previously collected from a subject to be diagnosed;
b) contacting the sample with the spike protein as cited in any of claims 1-11 and the ACE-2 protein as cited in any of claims 1-11; and
c) determining the level of interaction between the spike protein and the ACE-2;
wherein the reduction of the interaction level at the presence of the sample indicates the subject is infected or was infected with a SARS-CoV.

16. A method of preparing an inhibitor of the binding of a viral surface protein to a cell receptor comprising
a) identifying the inhibitor by performing the method of any of claims 1-10 or 13, and
b) isolating the inhibitor.

17. Kit comprising the cell of claim 10 together with a recombinant purified cell receptor protein.
